# EUROPEAN PATENT SPECIFICATION

(11) **EP 3 784 759 B1**
(45) Date of publication and mention of the grant of the patent: **17.07.2024**
(21) Application number: 19716956.8
(22) Date of filing: 16.04.2019
(51) Int. Cl.: C10G 33/08, B01D 17/02, B01D 17/12, G01N 33/28

(54) **SYSTEM AND METHOD FOR DRAINING A HYDROCARBON STORAGE TANK**
SYSTEM UND VERFAHREN ZUM ENTLEEREN EINES KOHLENWASSERSTOFFLAGERTANKS
SYSTÈME ET PROCÉDÉ DE PURGE D'UN RÉSERVOIR DE STOCKAGE D'HYDROCARBURES

(30) Priority: 24.04.2018 EP 18382278
(43) Date of publication of application: 03.03.2021
(73) Proprietor: Petroleos del Norte, S.A., 48550 Muskiz (Vizcaya) (ES)
(72) Inventor: ELEXPE ROVIRA, José María, 48550 Muskiz (Vizcaya) (ES)
(74) Representative: Balder IP Law, S.L.
(86) International application number: PCT/EP2019/059861
(87) International publication number: WO 2019/206749

(56) References cited:
- US-A- 3 565 252
- US-A- 3 966 602
- US-A- 4 596 136
- US-A- 5 139 653
- US-A1- 2014 083 950

## Description

### TECHNICAL FIELD

The present invention relates to the field of hydrocarbon storage tanks More specifically, the present invention relates to systems and methods for draining water from hydrocarbon storage tanks.

### BACKGROUND OF THE INVENTION

Hydrocarbons are extracted from hydrocarbon storage tanks when they must be processed or transported. However, water, which may have different origins, is often deposited into these storage tanks as well.

The existence of water in these tanks is undesirable since hydrocarbons are the only fluid material that should be extracted from the tanks for the processing or transport thereof.

Systems and methods for draining a hydrocarbon tank are known. These systems and methods aim to drain the part of the hydrocarbon tank content corresponding to water. To do so, in general it has a drainage duct or line with one end normally in the lower part of the tank (where the water is found) and another end outside of the tank to remove the water, and a valve in the drainage duct or line to allow or prevent drainage. One of the most basic methods used to carry out said drainage is by means of an operator who opens and closes the valve according to whether the fluid material that is being drained seems to be or contain water.

To try to improve both the efficiency and effectiveness of the draining process, automatic detection and drainage systems that make the presence of an operator during the draining process unnecessary have been developed in the state of the prior art.

Patent document US-5139653-A describes an automatic system for draining water from an oil storage tank. The system includes a recirculation line of fluid material, a pump for recirculating the fluid material, a detector, a drainage valve, and a logical unit that operates the drainage valve when the measurements of the detector indicate the existence of water in the fluid material.

Patent document US-3966602-A discloses processes and apparatuses for automatically restoring to usefulness spent cleaning and/or dying liquids comprising an aqueous phase and an oil phase with 1,1,2-thrichloro-1,2,2-trifluoroethane. The aqueous phase can be separated from the oil phase so that the oil phase can be regenerated.

Other developments of the prior art to automatize systems for separating substances are patent document US-2014083950-A1, which describes a method and apparatus for operating on skimmed oil/water picked up from a body of water to separate the oil from the water and discharge the oil-free water back into the body of water; patent document US-3565252-A, which describes a system for handling tank washings for oil tankers, means for separating oil from water are provided to assure that water passing overboard will not have present therein oil in excess of a predetermined concentration; or patent document US-4596136-A, which describes a method of determining the net volume of water and oil in a flow stream.

There is an interest in providing a system and a method for draining water from a hydrocarbon storage tank and prioritizing the non-drainage of hydrocarbons.

### DESCRIPTION OF THE INVENTION

A first aspect of the invention relates to a drainage system for a hydrocarbon storage tank as defined in claim 1, comprising: a recirculation duct adapted for the circulation of a fluid material, and also adapted to introduce a first end and a second end thereof into the hydrocarbon storage tank; a pump coupled to the recirculation duct and adapted to recirculate the fluid material in the recirculation duct so that it sucks in the fluid material in a first part of the recirculation duct and pushes the fluid material in a second part of the recirculation duct; a drainage duct adapted for the circulation of the fluid material, and also adapted to drain the fluid material through one end thereof; a motorized drain valve coupled to the drainage duct and adapted to allow and prevent drainage of the fluid material; at least one probe for detecting the presence of water in hydrocarbon; and a unit configured to receive measurements of the at least one probe; the unit is also configured to stop the pump and open the motorized drain valve to enable drainage when the received measurements of the at least one probe exceed an established threshold; the at least one probe is coupled to the first part of the recirculation duct; and the drainage duct is connected to the first part of the recirculation duct, and it is upstream from the pump and downstream from the at least one probe.

The system drains the fluid material coming from the hydrocarbon storage tank when the fluid material comprises water; thus, most or all of the material that remains in the storage tank is, precisely, hydrocarbon.

The pump recirculates the fluid liquid through the recirculation duct in a way that the fluid material passes from the tank to the first part of the recirculation duct by sucking, from the first part to the second part of the recirculation duct (through the pump), and from the second part once again to the tank (if it is not being drained) by impulsion.

Based on the detection carried out by the at least one probe, which measures (or which measure, in the case that the at least one probe is a plurality of probes) the ratio of fluid content (water/hydrocarbon) and based on the established threshold, the system drains (opens the drainage valve) the fluid material depending on whether it comprises more or less water. For this purpose, the at least one probe measures the water/hydrocarbon ratio of the fluid material, and the unit operates the system based on the configurations given to said unit depending on the ratio measured (said configurations could be established on the same unit or on a control panel connected to the unit). The unit is preferably a programmable logic controller or PLC. Although the hydrocarbons and the water have different densities and are immiscible, the fluid material that is recirculated by means of the pump can contain both hydrocarbon and water.

When the measurements received by the unit of each of the at least one probe corresponds to a positive detection of water (preferably a detection of at least a certain amount of water present in the fluid material) according to the established threshold, the unit stops the pump and opens the motorized drainage valve to drain the fluid material due to the water content thereof. Preferably, the first part of the recirculation duct is a part arranged horizontally.

The drainage of the fluid material through the drainage duct is carried out by pressure of a column of the fluid material in the recirculation duct and the drainage duct. Instead of pumping for drainage, the pressure generated by the column due to gravity is enough to carry out the drainage.

In some embodiments, the unit is also configured to close the motorized drainage valve to prevent drainage when the received measurements of at least one of the at least one probe do not exceed the established threshold.

When the measurements of at least one probe of the at least one probe (i.e. if the at least one probe comprises one single probe, then the measurements of said single probe; if the at least one probe comprises two or more probes, then the measurements of at least one of said two or more probes) do not correspond to a positive detection of water (preferably a detection of at least a certain amount of water present in the fluid material) according to the established threshold, the unit proceeds to close the motorized drainage valve if it is open (when it has been draining fluid material since it contains water) to prevent the fluid material with little or no water content from being drained. Subsequently, an operator can start the system again (for example, from a control center that is connected to the system) such that recirculation and drainage is carried out.

In some embodiments, the unit is also configured to start the pump to recirculate the fluid material in the recirculation duct when the received measurements of at least one of the at least one probe do not exceed the established threshold.

When the measurements of at least one probe of the at least one probe (i.e. if the at least one probe comprises one single probe, then the measurements of said single probe; if the at least one probe comprises two or more probes, then the measurements of at least one of said two or more probes) do not correspond to a positive detection of water (preferably a detection of at least a certain amount of water present in the fluid material) according to the established threshold, the unit proceeds to start the pump and thus recirculate fluid material, the motorized drainage valve being closed.

Since the unit can operate the system sequentially, such that, according to the received measurements of the at least one probe, it stops the pump and opens the motorized drainage valve, or it closes the motorized drainage valve and starts the pump, it progressively drains the fluid material stored in the tank as said sequence is repeated.

According to the first aspect of the invention, the recirculation duct comprises a first duct in the first part, the first duct comprising an end adapted for introduction into the tank; the recirculation duct further comprises a second duct in the second part, the second duct comprising an end adapted for introduction into the tank; the at least one probe comprises a first probe coupled to a part of the first duct, the part of the first duct being a part arranged horizontally.

The pump is coupled to the first and second ducts in a way that connects the first duct to the second duct. In this regard, the first duct is connected to the second duct through the pump allowing fluid communication from the first duct to the second duct.

The first duct acts as a suction line of the pump and makes it possible to extract fluid material from the tank so that said material circulates through the system and can be drained if necessary. The second duct acts as a drive line of the pump and makes it possible to introduce the fluid material circulated by the system into the tank; said fluid material is again introduced into the tank if it is not drained since the measured water/hydrocarbon ratio present in the fluid material corresponds to the fact that there is no water, or that the ratio of water is so low that it is preferable not to drain the fluid material (because a considerable amount of hydrocarbon would be drained).

The first probe detects the water/hydrocarbon ratio of the fluid material when it is introduced from the tank into the system, particularly before the fluid material reaches the point where the draining duct connects to the first duct.

The horizontal arrangement of the first part, in other words, a part of the first duct that is horizontal, makes it possible to have more efficient drainage since in laminar flow, the water of the fluid material is kept in the lower part of said horizontal part.

In some embodiments, the first probe is coupled to the part of the first duct at a first height; and the at least one probe further comprises a second probe coupled to the part of the first duct at a second height, the second height being greater than the first height.

The second probe provides complementary measurements to determine the ratio of water/hydrocarbon present in the fluid material, thereby allowing for more reliable drainage or non-drainage when determining the content of the fluid material in different parts thereof with greater accuracy. In this regard, providing the first and second probes at different heights is desirable since if it makes the fluid material circulate with a laminar flow, the water is kept in the lower part (due to the density thereof with respect to that of the hydrocarbon) and the hydrocarbon is kept in the upper part. With the probes at different heights, it is possible to establish the minimum volume of water that must exist in the part of the duct where the measurements are taken to carry out the drainage. The second probe is upstream from the pump.

When the at least one probe comprises two or more probes, since there are more measurements to consider, it also prevents draining of the hydrocarbon by mistake due to a possible loss of calibration of one of the probes (in other words, the fluid material contains little or no water).

In some embodiments, the second probe is coupled downstream from the first probe. In some embodiments, the first probe is coupled downstream from the second probe.

In some embodiments, the system further comprises a third probe, the third probe being coupled to the second duct; the unit is also configured to receive measurements of the third probe; and the unit is also configured to stop the pump and open the motorized drain valve to enable drainage when the received measurements of the at least one probe and the third probe exceed an established threshold. In these embodiments, the at least one probe at least comprises the first and second probes.

The measurements of the third probe coupled to the second duct (therefore, downstream from the pump), which are also complementary, serve to determine if while fluid material recirculates, a possible detection of water in said material (detection carried out by the first and second probes) is due to a possible water bag. In the case that the first and second probes (of the at least one probe) detect water, and the third probe also detects water, the unit operates the system to carry out the drainage upon determining that the presence of water is not due to a water bag.

During the draining process, the unit only uses the measurements of the at least one probe to determine whether the water/hydrocarbon ratio corresponds to hydrocarbon content, and therefore, the draining process should be stopped. In other words, the measurements of the third probe are not used by the unit for the purpose of determining when the draining process should be stopped.

In some embodiments, the third probe is coupled to a part of the second duct, the part of the second duct being a part arranged horizontally.

In some embodiments, the system further comprises the tank. In these embodiments, the first end of the recirculation duct is introduced into the tank; and the second end of the recirculation duct is introduced into the tank.

In some embodiments, the system further comprises a chamber. In these embodiments, the chamber is coupled to the drainage duct in fluid communication.

In some embodiments, the pump is adapted to recirculate the fluid material in the recirculation duct seeking to favor laminar flow. To this end, the generation of turbulence in the fluid material is reduced since the pump is adapted to recirculate the fluid material in the recirculation duct with a laminar flow.

In some embodiments, the pump is a positive displacement pump. In some embodiments, the pump is a positive displacement rotary pump. In some embodiments, the pump is a positive displacement rotary screw pump.

When the fluid material is recirculated in the recirculation duct, it is desirable to do so in such a way that it favors a laminar flow in the fluid material (and, therefore, reduces the generation of turbulence) at least in the first part of the recirculation duct (or the first duct, in the embodiments where the recirculation duct comprises the first duct in the first part). Since the connection to the drainage duct is found in the first part, the water in a laminar flow of the fluid material is kept in the lower part of the duct due to the greater density thereof, which makes it difficult to drain the possible hydrocarbon content in the fluid material during the draining operations.

A second aspect of the invention relates to a method for draining a hydrocarbon storage tank according to claim 7, comprising: pumping a fluid material of the hydrocarbon storage tank through a recirculation duct so that it sucks in the fluid material in a first part of the recirculation duct and pushes the fluid material in a second part of the recirculation duct; measuring an amount of water present in the fluid material in the recirculation duct with at least one probe for detecting the presence of water in hydrocarbon; stopping the pumping of the fluid material through the recirculation duct when the measurements of the at least one probe exceed an established threshold; and opening a motorized drainage valve to drain the fluid material through a drainage duct when the measurements of the at least one probe exceed an established threshold; measurement with the at least one probe is carried out in the first part of the recirculation duct; and the drainage duct is connected to the first part of the recirculation duct, and it is downstream from the at least one probe.

In the draining process of the hydrocarbon storage tank, fluid material of the tank is recirculated through the recirculation duct and, once inside, the water/hydrocarbon ratio of the fluid material is checked in order to proceed with draining or not.

When the measurements of the at least one probe are greater than the established threshold, it is determined that the water/hydrocarbon ratio in the fluid material is such that the fluid material should be drained. At this time, fluid material is no longer pumped through the recirculation duct in order to avoid reintroducing it into the tank since it contains water. The motorized drainage valve that allows the fluid material to be drained through the drainage duct is also opened, thereby eliminating part of the water from the tank that is deposited in it.

The pumping of the fluid material of the tank through the recirculation duct is carried out with a pump coupled to the recirculation duct and adapted to recirculate the fluid material in the recirculation duct. The drainage duct is upstream from the pump. Preferably, the first part of the recirculation duct is a part arranged horizontally.

The drainage of the fluid material through the drainage duct is carried out by pressure of a column of the fluid material in the recirculation duct and the drainage duct, thereby making pumping to carry out drainage unnecessary.

In some embodiments, the method further comprises closing the motorized drainage valve to prevent drainage when the measurements of at least one of the at least one probe do not exceed the established threshold.

When the measurement of at least one probe (i.e. if the at least one probe comprises one single probe, then the measurements of said single probe; if the at least one probe comprises two or more probes, then the measurements of at least one of said two or more probes) is not greater than the established threshold, it is determined that there is no water content in the fluid material or that the water content is not high enough to drain it. Therefore, the motorized drainage valve is closed.

In some embodiments, the method further comprises pumping the fluid material of the tank through the recirculation duct when the measurements of at least one of the at least one probe do not exceed the established threshold.

When the measurement of at least one probe (i.e. if the at least one probe comprises one single probe, then the measurements of said single probe; if the at least one probe comprises two or more probes, then the measurements of at least one of said two or more probes) is not greater than the established threshold, it is determined that there is no water content in the fluid material or that the water content is not high enough to drain it. Therefore, the fluid material of the tank is pumped again for the purpose of recirculating it and checking whether another part of the fluid material contains water for the drainage thereof.

According to the second aspect of the invention, the recirculation duct comprises a first duct in the first part, the first duct comprising an end adapted for introduction into the tank; the recirculation duct further comprises a second duct in the second part, the second duct comprising an end adapted for introduction into the tank; the at least one probe comprises a first probe; and the measurement with the first probe is carried out in a part of the first duct, the part of the first duct being a part arranged horizontally.

The pump is coupled to the first and second ducts in a way that connects the first duct to the second duct. In this regard, the first duct is connected to the second duct through the pump allowing fluid communication from the first duct to the second duct.

In some embodiments, the measurement with the first probe is carried out in the part of the first duct at a first height; the at least one probe further comprises a second probe; and the measurement with the second probe is carried out in the part of the first duct at a second height, the second height being greater than the first height.

In some embodiments, the method further comprises measuring an amount of water present in the fluid material in the second duct with a third probe for detecting the presence of water in hydrocarbon; and wherein stopping the pumping of the fluid material through the recirculation duct and opening a motorized drainage valve to drain the fluid material through the drainage duct is carried out when the measurements of the at least one probe and the third probe exceed an established threshold. In these embodiments, the at least one probe at least comprises the first and second probes.

In some embodiments, the measurement with the third probe is carried out in a part of the second duct, the part of the second duct being a part arranged horizontally.

In some embodiments, the method further comprises stopping the pumping of the fluid material through the recirculation duct when the pumping has started and stopped two or more times during an established period of intermittent operation. The established period of intermittent operation can be configured.

In some embodiments, the method further comprises stopping the pumping of the fluid material through the recirculation duct when it has been pumped continuously for an established period of continuous operation. The established period of continuous operation can be configured.

In some embodiments, a chamber is coupled to the drainage duct in fluid communication.

In some embodiments, the pump is adapted to recirculate the fluid material in the recirculation duct seeking to favor laminar flow. To this end, the generation of turbulence in the fluid material is reduced since the pump is adapted to recirculate the fluid material in the recirculation duct with a laminar flow.

In some embodiments, the pump is a positive displacement pump. In some embodiments, the pump is a positive displacement rotary pump. In some embodiments, the pump is a positive displacement rotary screw pump.

Advantages similar to those described with regard to the first aspect of the invention can also be applied to the second aspect of the invention.

### BRIEF DESCRIPTION OF THE DRAWINGS

To complement the description and for the purpose of aiding to better understand the features of the invention according to practical embodiments thereof, a set of figures is attached as an integral part of the description in which the following has been depicted with an illustrative and non-limiting character:
Figure 1 shows a system according to an embodiment of the invention as a diagram.
Figure 2 shows a system according to another embodiment of the invention as a diagram.
Figures 3A-3B show the system of Figure 2 in different modes of operation as a diagram.
Figure 4 shows a laminar flow of fluid material in a horizontal section of the recirculation duct where it is connected to the drainage duct as a diagram.

### DESCRIPTION OF EMBODIMENTS OF THE INVENTION

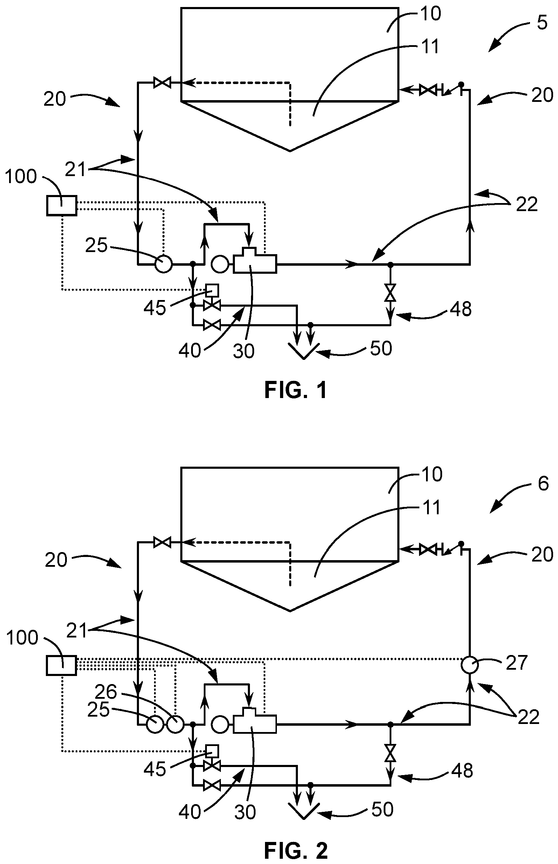

Figure 1 shows a system 5 according to an embodiment of the invention as a diagram.

The system 5 comprises a recirculation duct 20, a pump 30 coupled to the recirculation duct 20, a drainage duct 40, a motorized drainage valve 45, a probe 25 for detecting the presence of water in hydrocarbon, and a unit 100 configured to operate the system 5. The unit 100 is preferably a programmable logic controller, i.e. PLC.

The recirculation duct 20 includes a first end introduced into a hydrocarbon storage tank 10. The first end can be introduced into a lower part 11 of the tank 10 since it is normally where the water is deposited in the tank 10 due to the greater density thereof. The recirculation duct 20 includes a second end also introduced into the tank 10. The recirculation duct 20 comprises a first duct or line 21 (a first end of which corresponds to the first end of the recirculation duct 20) in a first part thereof, and a second duct or line 22 (a first end of which corresponds to the second end of the recirculation duct 20) in a second part thereof. The first duct or line 21 is connected to the second duct or line 22 through the pump 30.

The pump 30 enables a fluid material of the tank 10 to recirculate in the recirculation duct 20. The pump 30 is preferably a pump adapted to recirculate the fluid material in the recirculation duct, favoring a laminar flow, for example, a screw pump preferably with a low flow. While the pump 30 operates, it recirculates the fluid material in the following way: the pump 30 sucks the fluid material from the tank 10 (in some embodiments, it sucks the fluid material from the lower part 11 of the tank 10) to the first part of the recirculation duct 20 (in this example, the first duct or line 21); once in the first part, the pump 30 continues sucking the fluid material until it is transferred to the second part of the recirculation duct 20 (in this example, the second duct or line 22), and once in the second part, the pump 30 pushes the fluid material to re-introduce it into the tank 10.

When the unit 100 (as shown with dotted lines only for illustrative purposes) receives the measurements of the probe 25, which is coupled to the first part of the recirculation duct 20, and to a part of the first duct or line 21 arranged horizontally, it compares them to an established threshold to detect whether the fluid material that circulates through the recirculation duct 20 comprises water. In the case of positive detection of water (because the received measurements exceed the established threshold), the unit 100 stops the pump 30 and opens the motorized drainage valve 45 (as shown with dotted lines only for illustrative purposes) to drain the fluid material in a chamber 50. The unit 100 stops the pump 30 first and opens the motorized drainage valve 45 afterwards in order to reduce the probability of reintroducing the fluid material into the tank 10 and/or the probability of accidentally draining the fluid material that does not contain water. To this end, the pump 30 can provide the unit 100 with a signal that indicates the status of the pump 30 corresponding to whether it is on or off in order to check the operation thereof; the unit 100 can actuate the motorized drainage valve 45 when said signal indicates that the pump 30 is off.

The system 5 preferably comprises a non-return valve to prevent fluid material of the tank 10 from entering into the recirculation duct 20 through the second part of this duct (therefore, in the direction opposite the recirculation carried out by the pump 30). By operating the pump 30, it is possible to enable the recirculation of the fluid material as well as prevent it, which is in turn advantageous, since the recirculation can be blocked before opening the motorized drainage valve 45 and thereby prevent accidental drainage of fluid material with little or no water content.

In some examples, the system 5 further comprises a panel (not shown) connected to the unit 100; said panel includes one or more light indicators that allow an operator to check whether the detection of the probe 25 exceeds the established threshold, for example, for supervisory, monitoring and/or calibration tasks of the operations of the system 5 (for example, one task can be checking whether the tank 10 contains water or not without having the unit 100 open the motorized drainage valve 45 for drainage, even if the measurements of the probe 25 exceed the established threshold). To this end, that indicated on the one or more light indicators can be associated with the same established threshold with which the unit 100 operates the system 5, or associated with another established threshold, thereby indicating whether the measurements exceed the threshold. Likewise, the operator can carry out the draining process by means of the manual drainage duct 48, actuating the values that this duct 48 has.

The system 5 can also send information related to the operation of the system 5 wirelessly, for example, through a transmitter of the system 5. In this regard, the unit 100 can use the transmitter to send information to a control center, such as whether the system 5 has completed the drainage or whether it is carrying out automatic drainage, the status of the pump 30 (operating or not) and the status of the motorized drainage valve 45 (open or not), and even whether there is a malfunction in the system 5 that can prevent the automatic operation thereof (for example, failure in the pump 30, in the motorized drainage valve 45, in the probes 25-27, and/or in the unit 100 itself. If the transmitter is not able to send the information to the control center, an indication of failure can be activated in the signaling in the control center, although this does not prevent the system 5 from being able to continue functioning automatically. In the case that there is a malfunction in the system 5, the system 5 can be blocked so that it does not function automatically. In any case, the system 5 can be drained manually by means of the manual drainage duct 48.

In some examples, the system 5 further comprises the tank 10. In some of these examples and in some other examples, the system 5 further comprises the chamber 50.

In some examples, the unit 100 is also configured to stop the pump 30 when the pump 30 has started and stopped two or more times during an established period of intermittent operation.

The unit 100 can adjust the operation of the pump 30 to extend the useful life of the pump and of the motorized drainage valve. To this end, the unit 100 stops the pump 30 when during the established period of intermittent operation the pump 30 has started two or more times, and it has stopped two or more times. This usually occurs when fluid material is introduced into the system 5 sucking close to the water/hydrocarbon transition in the tank 10, such that the introduced fluid material contains water and hydrocarbon that greatly varies. Therefore, the pump 30 is stopped to prevent the wear, failure or breakdown thereof (as well as to prevent the wear, failure or breakdown of the motorized drainage valve 45) due to successive starts, since the intermittent operation also leads to inefficient draining.

The established period of intermittent operation can be configured. Likewise, the number of times that the pump must have started and stopped during the period of intermittent operation can also be configured.

In some examples, the unit 100 is also configured to stop the pump 30 when the pump 30 has been continuously on for an established period of continuous operation. The established period of continuous operation can be configured.

The unit 100 can adjust the operation of the pump 30 to maximize the safety of the draining process. To this end, the unit 100 stops the pump 30 when it has been continuously on (in other words, without having been stopped) for an established period of continuous operation.

Figure 2 shows a system 6 according to an embodiment of the invention as a diagram.

The system 6 of Figure 2 is similar to the system 5 of Figure 1, the difference mainly residing in the fact that the system 6 comprises, in addition to a first probe 25, a second probe 26 (coupled to the first part of the recirculation duct 20, preferably a part of the first duct or line 21 arranged horizontally) and a third probe 27 (coupled to the second part of the recirculation duct 20, and in this example, coupled to the second duct or line 22, preferably to a part of the second duct or line 22 arranged horizontally). The unit 100 receives measurements from the first, second and third probes 25, 26, 27 (as shown with dotted lines only for illustrative purposes) to operate the system 6.

When the pump 30 has been started, the fluid material of the tank 10 is recirculated through the recirculation duct 20 and the first, second and third probes 25, 26, 27 for detecting the presence of water in hydrocarbon detect whether there is water in the fluid material according to the threshold established.

When the measurements of the first, second and third probes 25, 26, 27 exceed the established threshold, the unit 100 stops the pump 30 and opens the motorized drainage valve 45. While it drains, the unit 100 takes into consideration the measurements of the first and second probes 25, 26 to determine when to stop the drainage: when the measurements of at least one probe of among the first and second probes 25, 26 do not exceed the established threshold, the unit 100 closes the motorized drainage valve 45; likewise, the unit can also start the pump 30 in this situation in order to recirculate fluid material again.

It is clear that in other examples systems and methods according to the present disclosure can include additional probes to provide more security thereto and thereby carry out drainage with better guarantees.

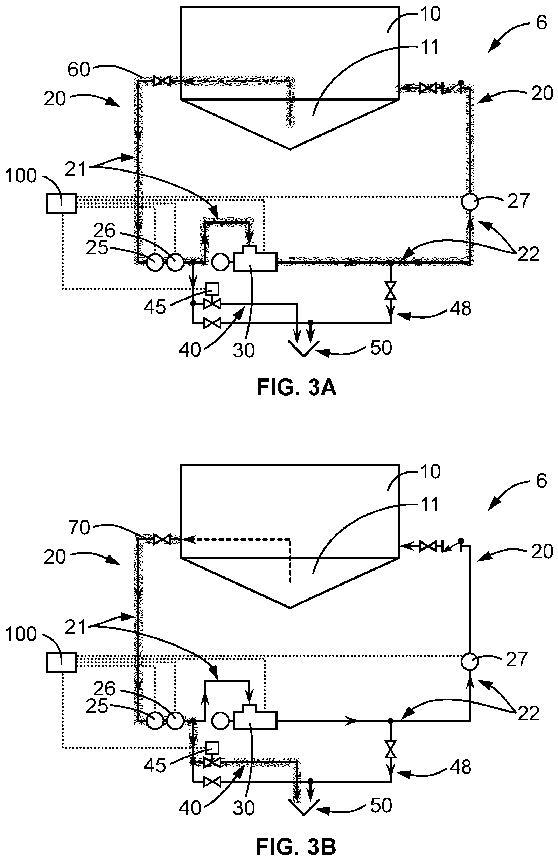

Figure 3A shows the system 6 of Figure 2 when it is recirculating 60 fluid material in the recirculation duct 20 as a diagram. The pump 30 operates and makes the fluid material recirculate 60 from the tank 10 to the same tank 10, first passing through the first duct or line 21, and then through the second duct or line 22.

During recirculation 60, the motorized drainage valve 45 is closed to prevent drainage of the fluid material to the chamber 50. Likewise, the first, second and third probes 25, 26, 27 measure the water/hydrocarbon ratio of the recirculated fluid material by means of which the unit 100 adjusts the operation of the system 6.

Figure 3B shows the system 6 of Figure 2 when it is draining 70 fluid material to the chamber 50 as a diagram. The motorized drainage valve 45 is open and drains 70 the fluid material towards the chamber 50.

During draining 70, the pump 30 does not operate, meaning that the fluid material is not recirculated in the recirculation duct 20. The column of fluid material (as shown with the reference 70) makes the pressure thereof cause drainage 70 to the chamber 50.

The unit 100 controls the draining process 70 by means of the measurements of the first and second probes 25, 26: when the measurements of one of these probes do not exceed the established threshold, the unit 100 closes the motorized drainage valve 45 to avoid draining fluid material with hydrocarbon content according to the configuration of the established threshold. Likewise, the unit 100 can re-start the pump 30 to once again recirculate fluid material of the tank 10, as explained with regard to Figure 3A. In this case, the unit 100 preferably closes the motorized drainage valve 45 first and then starts the pump 30 to prevent accidental drainage 70 of hydrocarbons. To this end, the motorized drainage valve 45 can provide the unit 100 with a signal indicating the status of the motorized drainage valve 45 corresponding to whether it is open or closed in order to check the operation thereof; the unit 100 can actuate the pump 30 when said signal indicates that the motorized drainage valve 45 is closed.

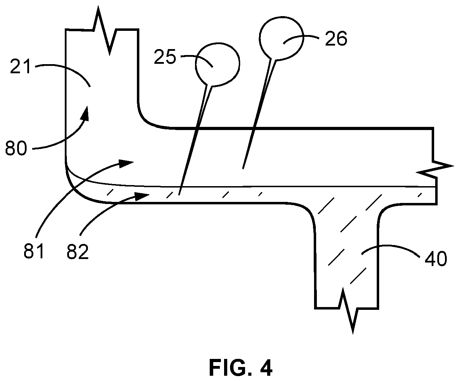

Figure 4 shows a laminar flow of fluid material 80 in a recirculation duct as a diagram. This figure partially shows a first duct or line 21 of the recirculation duct with a vertical section through which the fluid material 80 is collected (which contains hydrocarbon 81 and water 82) from a hydrocarbon storage tank, and a horizontal section. The figure also partially shows a drainage duct 40 connected to the first duct or line 21 (in the horizontal section), particularly a lower generatrix thereof 21.

The recirculation of the fluid material 80 is carried out so that a laminar flow is produced with the hydrocarbon 81 in one part and the water 82 in another part. Due to the difference in densities, the water 82 remains in the lower part of the laminar flow, such that during the draining process (when a motorized drainage valve or a manual drainage valve, in the case that an operator wishes to manually drain the tank, is opened), the part corresponding to the water 82 is drained.

First and second probes 25, 26 for detecting the presence of water in hydrocarbon are coupled to the first duct or line 21 and measure the presence of water 82 in the fluid material 80. The first and second probes 25, 26 are at different heights of the first duct or line 21 to enable a more accurate identification of the separation between hydrocarbon 81 and water 82 of the laminar flow. In other examples, the first and second probes 25, 26 are at the same height or at a height similar to that of the first duct or line 21. In this example, the first probe 25 is coupled to a point of the first duct or line 21 upstream from the point of the first duct or line 21 to which the second probe 26 is coupled. In another example that is not shown, the second probe 26 is coupled to a point of the first duct or line 21 upstream from the point of the first duct or line 21 to which the first probe 25 is coupled.

The drainage duct 40 is upstream from the pump of the system and downstream from the points to which the first and second probes 25, 26 are coupled.

Although the systems and methods of the present disclosure use one or more probes for detecting the presence of water in hydrocarbon, similar systems and methods in which probes for detecting the presence of hydrocarbon in fluid material (for example, water) can be used but are not claimed. As is evident to a person skilled in the art, the systems and methods that use probes for detecting the presence of hydrocarbon in the fluid material should be configured to carry out recirculation and drainage operations (in other words, operating the pump and the motorized drainage valve), so that: in the case of detecting hydrocarbon in the fluid material according to an established threshold, said fluid material is recirculated; and in the case that hydrocarbon is not detected in the fluid material according to the established threshold, said fluid material is drained.

In this text, the word "comprises" and its variants (such as "comprising", etc.) should not be understood in an exclusive sense, i.e. they do not exclude the possibility of that which is described including other elements, steps, etc.

Furthermore, the invention is not limited to the specific embodiments described herein, but rather encompasses the variations that one skilled in the art could make (e.g. in terms of choice of materials, dimensions, components, design, etc.), within the scope of the invention as defined in the appended claims.

## Claims

1. A draining system (5, 6) for a hydrocarbon (81) storage tank (10), comprising:
a recirculation duct (20) adapted for the circulation of a fluid material (80), and also adapted to introduce a first end and a second end thereof into the hydrocarbon (81) storage tank (10);
a pump (30) coupled to the recirculation duct (20) and adapted to recirculate (60) the fluid material (80) in the recirculation duct (20) so that sucks in the fluid material (80) in a first part of the recirculation duct (20) and pushes the fluid material (80) in a second part of the recirculation duct (20);
a drainage duct (40) adapted for the circulation of the fluid material (80), and also adapted to drain (70) the fluid material (80) through one end thereof;
a motorized drain valve (45) coupled to the drainage duct (40) and adapted to allow and prevent drainage (70) of the fluid material (80);
at least one probe (25, 26) for detecting the presence of water (82) in hydrocarbon (81); and
a unit (100) configured to receive measurements of the at least one probe (25, 26);
wherein the unit (100) is also configured to stop the pump (30) and open the motorized drain valve (45) to enable drainage (70) when the received measurements of the at least one probe (25, 26) exceed an established threshold, the unit (100) being configured to stop the pump (30) first and open the motorized drain valve (45) afterwards;
wherein the drainage duct (40) is connected to the first part of the recirculation duct (20), and it is upstream from the pump (30) and downstream from the at least one probe (25, 26);
wherein the recirculation duct (20) and the drainage duct (40) are arranged so as to drain (70) the fluid material (80) through the drainage duct (40) by pressure of a column of the fluid material (80) in the recirculation duct (20) and the drainage duct (40);
wherein the recirculation duct (20) comprises:
a first duct (21) in the first part, the first duct (21) comprising an end adapted for introduction into the tank (10); and
a second duct (22) in the second part, the second duct (22) comprising an end adapted for introduction into the tank (10); and
wherein the at least one probe (25, 26) comprises a first probe (25) coupled to a part of the first duct (21), the part of the first duct (21) being a part arranged horizontally.

2. The system (5, 6) according to claim 1, wherein the unit (100) is also configured to close the motorized drainage valve (45) to prevent drainage (70) when the received measurements of at least one of the at least one probe (25, 26) do not exceed the established threshold.

3. The system (5, 6) according to claim 2, wherein the unit (100) is also configured to start the pump (30) to recirculate (60) the fluid material (80) in the recirculation duct (20) when the received measurements of at least one of the at least one probe (25, 26) do not exceed the established threshold.

4. The system (6) according to any of claims 1-3, wherein:
the first probe (25) is coupled to the part of the first duct (21) at a first height; and
the at least one probe (25, 26) further comprises a second probe (26) coupled to the part of the first duct (21) at a second height, the second height being greater than the first height.

5. The system (6) according to claim 4, which further comprises a third probe (27), the third probe (27) being coupled to the second duct (22); wherein the unit (100) is also configured to receive measurements of the third probe (27); and wherein the unit (100) is also configured to stop the pump (30) and open the motorized drain valve (45) to enable drainage (70) when the received measurements of the at least one probe (25, 26) and the third probe (27) exceed an established threshold.

6. The system (5, 6) according to any of the preceding claims, wherein the pump (30) is a positive displacement pump.

7. A method for draining a hydrocarbon (81) storage tank (10), comprising:
pumping (60) a fluid material (80) of the hydrocarbon (81) storage tank (10) through a recirculation duct (20) so that it sucks in the fluid material (80) in a first part of the recirculation duct (20) and pushes the fluid material (80) in a second part of the recirculation duct (20), wherein the pumping (60) is carried out with a pump (30) coupled to the recirculation duct (20);
measuring an amount of water (82) present in the fluid material (80) in the recirculation duct (20) with at least one probe (25, 26) for detecting the presence of water (82) in hydrocarbon (81);
stopping the pumping (60) of the fluid material (80) through the recirculation duct (20) when the measurements of the at least one probe (25, 26) exceed an established threshold; and
opening a motorized drainage valve (45) to drain (70) the fluid material (80) through a drainage duct (40) both when the measurements of the at least one probe (25, 26) exceed an established threshold and after the step of stopping the pumping (60);
wherein the drainage duct (40) is connected to the first part of the recirculation duct (20), and it is downstream from the at least one probe (25, 26);
wherein the drainage (70) of the fluid material (80) through the drainage duct (40) is carried out by pressure of a column of the fluid material (80) in the recirculation duct (20) and the drainage duct (40);
wherein the recirculation duct (20) comprises in the first part a first duct (21) with an end introduced into the tank (10), and in the second part a second duct (22) with an end introduced into the tank (10);
wherein the at least one probe (25, 26) comprises a first probe (25); and
wherein the measurement with the first probe (25) is carried out in a part of the first duct (21), the part of the first duct (21) being a part arranged horizontally.

8. The method according to claim 7, which further comprises closing the motorized drainage valve (45) to prevent drainage (70) when the received measurements of at least one of the at least one probe (25, 26) do not exceed the established threshold.

9. The method according to claim 8, which further comprises pumping (60) the fluid material (80) of the tank (10) through the recirculation duct (20) when the measurements of at least one of the at least one probe (25, 26) do not exceed the established threshold.

10. The method according to any of claims 7-9, wherein:
the measurement with the first probe (25) is carried out in the part of the first duct (21) at a first height;
the at least one probe (25, 26) further comprises a second probe (26); and
the measurement with the second probe (26) is carried out in the part of the first duct (21) at a second height, the second height being greater than the first height.

11. The method according to claim 10, which further comprises measuring an amount of water (82) present in the fluid material (80) in the second duct (22) with a third probe (27) for detecting the presence of water (82) in hydrocarbon (81); and wherein stopping the pumping (60) of the fluid material (80) through the recirculation duct (20) and opening a motorized drainage valve (45) to drain (70) the fluid material (80) through the drainage duct (40) is carried out when the measurements of the at least one probe (25, 26) and the third probe (27) exceed an established threshold.

12. The method according to claim 7, wherein the pump (30) is a positive displacement pump.

## Patentansprüche

1. Ein Entleerungssystem (5, 6) für einen Speichertank (10) für Kohlenwasserstoff (81), umfassend:
eine Rezirkulationsleitung (20), die für die Zirkulation eines fluiden Materials (80) eingerichtet ist und auch dazu eingerichtet ist, ein erstes Ende und ein zweites Ende davon in den Speichertank (10) für Kohlenwasserstoff (81) einzuführen;
eine Pumpe (30), die mit der Rezirkulationsleitung (20) gekoppelt und eingerichtet ist, das fluide Material (80) in der Rezirkulationsleitung (20) zu rezirkulieren (60), so dass es in dem fluiden Material (80) in einem ersten Teil der Rezirkulationsleitung (20) saugt und das fluide Material (80) in einen zweiten Teil der Rezirkulationsleitung (20) drückt;
eine Drainageleitung (40), die für die Zirkulation des fluiden Materials (80) eingerichtet ist und auch dazu eingerichtet ist, das fluide Material (80) durch ein Ende davon abzulassen (70);
ein motorisiertes Ablassventil (45), das mit der Drainageleitung (40) verbunden und dazu eingerichtet ist, ein Ablassen (70) des fluiden Materials (80) zuzulassen und zu verhindern;
mindestens eine Sonde (25, 26) zum Nachweisen des Vorhandenseins von Wasser (82) in Kohlenwasserstoff (81); und
eine Einheit (100), die dazu konfiguriert ist, Messungen der mindestens einen Sonde (25, 26) zu empfangen;
wobei die Einheit (100) auch dazu konfiguriert ist, die Pumpe (30) zu stoppen und das motorisierte Ablassventil (45) zu öffnen, um ein Ablassen (70) zu ermöglichen, wenn die empfangenen Messungen der mindestens einen Sonde (25, 26) einen festgelegten Schwellenwert überschreiten, wobei die Einheit (100) dazu konfiguriert ist, zuerst die Pumpe (30) zu stoppen und danach das motorisierte Ablassventil (45) zu öffnen;
wobei die Drainageleitung (40) mit dem ersten Teil der Rezirkulationsleitung (20) verbunden ist und sich stromaufwärts der Pumpe (30) und stromabwärts der mindestens einen Sonde (25, 26) befindet;
wobei die Rezirkulationsleitung (20) und die Drainageleitung (40) so angeordnet sind, dass das fluide Material (80) durch die Drainageleitung (40) mittels des Drucks einer Säule des fluiden Materials (80) in der Rezirkulationsleitung (20) und der Drainageleitung (40) abgeleitet wird;
wobei die Rezirkulationsleitung (20) umfasst:
eine erste Leitung (21) im ersten Teil, wobei die erste Leitung (21) ein zum Einführen in den Tank (10) eingerichtetes Ende aufweist; und
eine zweite Leitung (22) im zweiten Teil, wobei die zweite Leitung (22) ein zum Einführen in den Tank (10) eingerichtetes Ende aufweist; und
wobei die mindestens eine Sonde (25, 26) eine erste Sonde (25) umfasst, die mit einem Teil der ersten Leitung (21) verbunden ist, wobei der Teil der ersten Leitung (21) ein horizontal angeordneter Teil ist.

2. System (5, 6) nach Anspruch 1, wobei die Einheit (100) auch dazu konfiguriert ist, das motorisierte Ablassventil (45) zu schließen, um ein Ablassen (70) zu verhindern, wenn die empfangenen Messungen von mindestens einer der mindestens einen Sonde (25, 26) den festgelegten Schwellenwert nicht überschreiten.

3. System (5, 6) nach Anspruch 2, wobei die Einheit (100) auch dazu konfiguriert ist, die Pumpe (30) zu starten, um das fluide Material (80) in die Rezirkulationsleitung (20) zurückzuleiten leiten (60), wenn die empfangenen Messungen von mindestens einer der mindestens einen Sonde (25, 26) den festgelegten Schwellenwert nicht überschreiten.

4. Das System (6) nach einem der Ansprüche 1-3, wobei:
die erste Sonde (25) mit dem Teil der ersten Leitung (21) in einer ersten Höhe verbunden ist; und
die mindestens eine Sonde (25, 26) außerdem eine zweite Sonde (26) aufweist, die mit dem Teil der ersten Leitung (21) in einer zweiten Höhe verbunden ist, wobei die zweite Höhe größer ist als die erste Höhe.

5. System (6) nach Anspruch 4, das ferner eine dritte Sonde (27) umfasst, wobei die dritte Sonde (27) mit der zweiten Leitung (22) gekoppelt ist; wobei die Einheit (100) auch dazu konfiguriert ist, Messungen der dritten Sonde zu empfangen (27); und wobei die Einheit (100) auch dazu konfiguriert ist, die Pumpe (30) zu stoppen und das motorisierte Ablassventil (45) zu öffnen, um das Ablassen (70) zu ermöglichen, wenn die empfangenen Messungen der mindestens einen Sonde (25, 26) und der dritten Sonde (27) einen festgelegten Schwellenwert überschreiten.

6. Das System (5, 6) nach einem der vorhergehenden Ansprüche, wobei die Pumpe (30) eine Verdrängerpumpe ist.

7. Verfahren zum Entleeren eines Speichertank (10) für Kohlenwasserstoff (81), umfassend:
Pumpen (60) eines fluiden Materials (80) aus dem Speichertank (10) für Kohlenwasserstoff (81) durch eine Rezirkulationsleitung (20), so dass das fluide Material (80) in einem ersten Teil der Rezirkulationsleitung (20) angesaugt und das fluide Material (80) in einen zweiten Teil der Rezirkulationsleitung (20) gedrückt wird, wobei das Pumpen (60) mit einer mit der Rezirkulationsleitung (20) verbundenen Pumpe (30) ausgeführt wird;
Messen einer in dem fluiden Material (80) in der Rezirkulationsleitung (20) vorhandenen Wassermenge (82) mit mindestens einer Sonde (25, 26) zum Nachweisen des Vorhandenseins von Wasser (82) in Kohlenwasserstoff (81);
Anhalten des Pumpens (60) des fluiden Materials (80) durch die Rezirkulationsleitung (20), wenn die Messungen der mindestens einen Sonde (25, 26) einen festgelegten Schwellenwert überschreiten; und
Öffnen eines motorisierten Ablassventils (45) zum Ablassen (70) des fluiden Materials (80) durch eine Drainageleitung (40) sowohl, wenn die Messungen der mindestens einen Sonde (25, 26) einen festgelegten Schwellenwert überschreiten, als auch nach dem Schritt des Anhaltens des Pumpens (60);
wobei die Drainageleitung (40) mit dem ersten Teil der Rezirkulationsleitung (20) verbunden ist und sich stromabwärts von der mindestens einen Sonde (25, 26) befindet
wobei das Ablassen (70) des fluiden Materials (80) durch die Drainageleitung (40) durch den Druck einer Säule des fluiden Materials (80) in der Rezirkulationsleitung (20) und der Drainageleitung (40) erfolgt;
wobei die Rezirkulationsleitung (20) im ersten Teil eine erste Leitung (21) mit einem in den Tank (10) eingeführten Ende und im zweiten Teil eine zweite Leitung (22) mit einem in den Tank (10) eingeführten Ende aufweist;
wobei die mindestens eine Sonde (25, 26) eine erste Sonde (25) umfasst; und
wobei die Messung mit der ersten Sonde (25) in einem Teil der ersten Leitung (21)stattfindet, wobei der Teil der ersten Leitung (21) ein Teil ist, der horizontal verläuft.

8. Verfahren nach Anspruch 7, das außerdem das Schließen des motorisierten Ablassventils (45) aufweist, um ein Ablassen (70) zu verhindert, wenn die empfangenen Messungen von mindestens einer der mindestens einen Sonde (25, 26) den festgelegten Schwellenwert nicht überschreiten.

9. Verfahren nach Anspruch 8, das außerdem das Pumpen (60) des fluiden Materials (80) aus dem Tank (10) durch die Rezirkulationsleitung (20) aufweist, wenn die Messungen mindestens einer der mindestens einen Sonde (25, 26) den festgelegten Schwellenwert nicht überschreiten.

10. Das Verfahren nach einem der Ansprüche 7-9, wobei:
die Messung mit der ersten Sonde (25) in dem Teil der ersten Leitung (21) in einer ersten Höhe ausgeführt wird;
die mindestens eine Sonde (25, 26) außerdem eine zweite Sonde (26) aufweist; und
die Messung mit der zweiten Sonde (26) in dem Teil des der ersten Leitung (21) in einer zweiten Höhe ausgeführt wird, wobei die zweite Höhe größer ist als die erste Höhe.

11. Verfahren nach Anspruch 10, das ferner das Messen einer in dem fluiden Material (80) in der zweiten Leitung (22) vorhandenen Wassermenge (82) mit einer dritten Sonde (27) zum Nachweis des Vorhandenseins von Wasser (82) in Kohlenwasserstoff (81) aufweist, und wobei das Stoppen des Pumpens (60) des fluiden Materials (80) durch die Rezirkulationsleitung (20) und das Öffnen eines motorisierten Ablassventils (45) zum Ablassen (70) des fluiden Materials (80) durch die Drainageleitung (40) ausgeführt wird, wenn die Messungen der mindestens einen Sonde (25, 26) und der dritten Sonde (27) einen festgelegten Schwellenwert überschreiten.

12. Verfahren n ach Anspruch 7, wobei die Pumpe (30) eine Verdrängerpumpe ist.

## Revendications

1. Système de drainage (5, 6) pour un réservoir de stockage (10) d'hydrocarbures (81), comprenant :
un conduit de recirculation (20) adapté à la circulation d'une matière fluide (80), et également adapté à la pénétration d'une première extrémité et d'une deuxième extrémité de celui-ci dans le réservoir de stockage (10) d'hydrocarbures (81) ;
une pompe (30) couplée au conduit de recirculation (20) et adaptée pour faire recirculer (60) la matière fluide (80) dans le conduit de recirculation (20) de manière à aspirer la matière fluide (80) dans une première partie du conduit de recirculation (20) et à refouler la matière fluide (80) dans une deuxième partie du conduit de recirculation (20) ;
un conduit de drainage (40) adapté à la circulation de la matière fluide (80), et également adapté pour drainer (70) la matière fluide (80) à travers l'une de ses extrémités ;
une vanne de drainage motorisée (45) couplée au conduit de drainage (40) et adaptée pour permettre et empêcher le drainage (70) de la matière fluide (80) ;
au moins une sonde (25, 26) pour détecter la présence d'eau (82) dans l'hydrocarbure (81) ; et
une unité (100) configurée pour recevoir les mesures de ladite au moins une sonde (25, 26) ;
l'unité (100) étant également configurée pour arrêter la pompe (30) et ouvrir la vanne de drainage motorisée (45) pour permettre le drainage (70) lorsque les mesures reçues de ladite au moins une sonde (25, 26) dépassent un seuil établi, l'unité (100) étant configurée pour d'abord arrêter la pompe (30) et ensuite ouvrir la vanne de drainage motorisée (45) ;
le conduit de drainage (40) étant relié à la première partie du conduit de recirculation (20), et se trouvant en amont de la pompe (30) et en aval de ladite au moins une sonde (25, 26) ;
le conduit de recirculation (20) et le conduit de drainage (40) étant disposés de manière à drainer (70) la matière fluide (80) à travers le conduit de drainage (40) par la pression d'une colonne de matière fluide (80) dans le conduit de recirculation (20) et le conduit de drainage (40) ;
le conduit de recirculation (20) comprenant :
un premier conduit (21) dans la première partie, le premier conduit (21) comprenant une extrémité adaptée à pénétrer dans le réservoir (10) ; et
un deuxième conduit (22) dans la deuxième partie, le deuxième conduit (22) comprenant une extrémité adaptée à pénétrer dans le réservoir (10) ; et
ladite au moins une sonde (25, 26) comprenant une première sonde (25) couplée à une partie du premier conduit (21), la partie du premier conduit (21) étant une partie disposée horizontalement.

2. Système (5, 6) selon la revendication 1, dans lequel l'unité (100) est également configurée pour fermer la vanne de drainage motorisée (45) afin d'empêcher le drainage (70) lorsque les mesures reçues d'au moins une desdites au moins une sonde (25, 26) ne dépassent pas le seuil établi.

3. Système (5, 6) selon la revendication 2, dans lequel l'unité (100) est également configurée pour démarrer la pompe (30) pour faire recirculer (60) la matière fluide (80) dans le conduit de recirculation (20) lorsque les mesures reçues d'au moins une desdites au moins une sonde (25, 26) ne dépassent pas le seuil établi.

4. Système (6) selon l'une quelconque des revendications 1 à 3, dans lequel :
la première sonde (25) est couplée à la partie du premier conduit (21) à une première hauteur ; et
ladite au moins une sonde (25, 26) comprend en outre une deuxième sonde (26) couplée à la partie du premier conduit (21) à une deuxième hauteur, la deuxième hauteur étant supérieure à la première hauteur.

5. Système (6) selon la revendication 4, qui comprend en outre une troisième sonde (27), la troisième sonde (27) étant couplée au deuxième conduit (22) ; l'unité (100) étant également configurée pour recevoir des mesures de la troisième sonde (27) ; et l'unité (100) étant également configurée pour arrêter la pompe (30) et ouvrir la vanne de drainage motorisée (45) pour permettre le drainage (70) lorsque les mesures reçues de ladite au moins une sonde (25, 26) et de la troisième sonde (27) dépassent un seuil établi.

6. Système (5, 6) selon l'une quelconque des revendications précédentes, dans lequel la pompe (30) est une pompe volumétrique.

7. Procédé de drainage d'un réservoir de stockage (10) d'hydrocarbures (81), comprenant :
le pompage (60) d'une matière fluide (80) du réservoir de stockage (10) d'hydrocarbures (81) à travers un conduit de recirculation (20) de manière à aspirer la matière fluide (80) dans une première partie du conduit de recirculation (20) et à refouler la matière fluide (80) dans une deuxième partie du conduit de recirculation (20), le pompage (60) étant effectué avec une pompe (30) couplée au conduit de recirculation (20) ;
la mesure d'une quantité d'eau (82) présente dans la matière fluide (80) dans le conduit de recirculation (20) à l'aide d'au moins une sonde (25, 26) pour détecter la présence d'eau (82) dans l'hydrocarbure (81) ;
l'arrêt du pompage (60) de la matière fluide (80) dans le conduit de recirculation (20) lorsque les mesures de ladite au moins une sonde (25, 26) dépassent un seuil établi ; et
l'ouverture d'une vanne de drainage motorisée (45) pour drainer (70) la matière fluide (80) à travers un conduit de drainage (40) à la fois lorsque les mesures de ladite au moins une sonde (25, 26) dépassent un seuil établi et après l'étape d'arrêt du pompage (60) ;
le conduit de drainage (40) étant relié à la première partie du conduit de recirculation (20), et se trouvant en aval de ladite au moins une sonde (25, 26) ;
le drainage (70) de la matière fluide (80) à travers le conduit de drainage (40) étant effectué par la pression d'une colonne de matière fluide (80) dans le conduit de recirculation (20) et le conduit de drainage (40) ;
le conduit de recirculation (20) comprenant dans la première partie un premier conduit (21) avec une extrémité pénétrant dans le réservoir (10), et dans la deuxième partie un deuxième conduit (22) avec une extrémité pénétrant dans le réservoir (10) ;
ladite au moins une sonde (25, 26) comprenant une première sonde (25) ; et
la mesure avec la première sonde (25) étant effectuée dans une partie du premier conduit (21), la partie du premier conduit (21) étant une partie disposée horizontalement.

8. Procédé selon la revendication 7, qui comprend en outre la fermeture de la vanne motorisée de drainage (45) pour empêcher le drainage (70) lorsque les mesures reçues de ladite au moins une sonde (25, 26) ne dépassent pas le seuil établi.

9. Procédé selon la revendication 8, qui comprend en outre le pompage (60) de la matière fluide (80) du réservoir (10) à travers le conduit de recirculation (20) lorsque les mesures d'au moins une desdites au moins une sonde (25, 26) ne dépassent pas le seuil établi.

10. Procédé selon l'une quelconque des revendications 7 à 9, dans lequel :
la mesure avec la première sonde (25) est effectuée dans la partie du premier conduit (21) à une première hauteur ;
ladite au moins une sonde (25, 26) comprend en outre une deuxième sonde (26) ; et
la mesure avec la deuxième sonde (26) est effectuée dans la partie du premier conduit (21) à une deuxième hauteur, la deuxième hauteur étant supérieure à la première hauteur.

11. Procédé selon la revendication 10, qui comprend en outre la mesure d'une quantité d'eau (82) présente dans la matière fluide (80) dans le deuxième conduit (22) avec une troisième sonde (27) pour détecter la présence d'eau (82) dans l'hydrocarbure (81) ; et l'arrêt du pompage (60) de la matière fluide (80) à travers le conduit de recirculation (20) et l'ouverture d'une vanne de drainage motorisée (45) pour drainer (70) la matière fluide (80) à travers le conduit de drainage (40) étant effectués lorsque les mesures de ladite au moins une sonde (25, 26) et de la troisième sonde (27) dépassent un seuil établi.

12. Procédé selon la revendication 7, dans lequel la pompe (30) est une pompe volumétrique.
